# FASCICULE DE BREVET EUROPEEN

(11) **EP 4 452 526 B1**
(45) Date de publication et mention de la délivrance du brevet: **10.06.2026**
(21) Numéro de dépôt: 22823606.3
(22) Date de dépôt: 07.12.2022
(51) Int. Cl.: B06B 1/06, A61B 8/00

(54) **DISPOSITIF D'IMAGERIE ULTRASONORE**
ULTRASCHALLBILDGEBUNGSVORRICHTUNG
ULTRASOUND IMAGING DEVICE

(30) Priorité: 21.12.2021 FR 2114109
(43) Date de publication de la demande: 30.10.2024
(73) Titulaire: Moduleus, 37100 Tours (FR)
(72) Inventeur: JEANNE, Edgard, 37100 TOURS (FR); ORDRONNEAU, Lucie, 37100 TOURS (FR); ROY, Mathieu, 37100 TOURS (FR)
(74) Mandataire: Cabinet Beaumont
(86) Numéro de dépôt international: PCT/EP2022/084702
(87) Numéro de publication internationale: WO 2023/117415

(56) Documents cités:
- EP-A1- 2 441 492
- EP-A1- 2 992 829
- FR-A1- 2 963 430
- US-A1- 2004 171 970
- US-A1- 2020 121 298
- US-A1- 2021 124 044

## Description

La présente demande est basée sur, et revendique la priorité de, la demande de brevet français FR2114109 déposée le 21 décembre 2021 et ayant pour titre "Dispositif d'imagerie Ultrasonore" qui est considérée comme faisant partie intégrante de la présente description dans les limites prévues par la loi.

### Domaine technique

La présente description concerne le domaine des dispositifs d'imagerie ultrasonore, et vise plus particulièrement des dispositifs d'acquisition d'empreintes de peau et/ou des dispositifs d'imagerie microvasculaire à base de transducteurs ultrasonores.

### Technique antérieure

Un dispositif d'imagerie ultrasonore comprend classiquement une pluralité de transducteurs ultrasonores, et un circuit électronique de commande connecté aux transducteurs. En fonctionnement, l'ensemble des transducteurs est disposé face à un objet ou corps dont on souhaite acquérir une image. Le circuit électronique de commande est configuré pour appliquer des signaux électriques d'excitation aux transducteurs, de façon à provoquer l'émission d'ondes ultrasonores par les transducteurs, en direction du corps à analyser. Les ondes ultrasonores émises par les transducteurs sont réfléchies par le corps à analyser (par sa structure interne et/ou superficielle), puis reviennent vers les transducteurs qui les convertissent à nouveau en signaux électriques. Ces signaux électriques de réponse sont lus par le circuit électronique de commande, et peuvent être mémorisés et analysés pour en déduire des informations sur le corps étudié.

Il serait souhaitable d'améliorer au moins en partie certains aspects des dispositifs d'imagerie ultrasonore connus tel que celui décrit dans le document US2020121298A1.

### Résumé de l'invention

Pour cela, un mode de réalisation prévoit un dispositif d'imagerie ultrasonore comportant un ensemble de transducteurs ultrasonores, une couche de couplage acoustique revêtant ledit ensemble de transducteurs ultrasonores, et un dispositif de chauffe adapté à chauffer la couche de couplage acoustique et un objet à imager disposé sur la couche de couplage acoustique lors d'une phase d'acquisition d'une image ultrasonore.

Selon un mode de réalisation, le dispositif comprend en outre un circuit électronique d'alimentation et de contrôle.

Selon un mode de réalisation, le dispositif de chauffe est adapté à récupérer de la chaleur générée par le circuit électronique d'alimentation et de contrôle et à dissiper tout ou partie de cette chaleur dans la couche de couplage acoustique, au-dessus de l'ensemble de transducteurs ultrasonores.

Selon un mode de réalisation, le dispositif de chauffe comprend une ou plusieurs feuilles métalliques agencées pour conduire la chaleur générée par le circuit électronique d'alimentation et de contrôle et dissiper tout ou partie de cette chaleur dans la couche de couplage acoustique, au-dessus de l'ensemble de transducteurs ultrasonores.

Selon un mode de réalisation, le dispositif de chauffe comprend en outre un ou plusieurs interrupteurs configurés pour interrompre le transfert de chaleur vers la couche de couplage acoustique si une température interne du dispositif excède un seuil prédéterminé.

Selon un mode de réalisation, le dispositif de chauffe comprend un module Peltier flexible dont une face froide est tournée vers le circuit électronique d'alimentation et de contrôle et dont une face chaude est tournée vers l'objet à imager.

Selon un mode de réalisation, le dispositif de chauffe comprend un tapis de chauffe comprenant une résistance métallique en serpentin, disposé entre l'ensemble de transducteurs ultrasonores et l'objet à imager.

Selon un mode de réalisation, le tapis de chauffe est noyé dans la couche de couplage acoustique.

Selon un mode de réalisation, le tapis de chauffe recouvre la couche de couplage acoustique.

Selon un mode de réalisation, le dispositif de chauffe comprend une couche chauffante en un matériau polymère thermoélectrique, intégrée à la couche de couplage acoustique.

Selon un mode de réalisation, la couche chauffante est connectée électriquement au circuit électronique d'alimentation et de contrôle par des éléments de connexion.

Selon un mode de réalisation, les transducteurs ultrasonores de l'ensemble de transducteurs ultrasonores sont des transducteurs CMUT ou PMUT, des transducteurs piézoélectriques ou piézocomposites, ou des transducteurs monocristal.

Selon un mode de réalisation, la couche de couplage acoustique comprend au moins une couche en un matériau polymère activable configuré pour présenter un premier module de Young lors d'une phase d'acquisition d'une image ultrasonore et un deuxième module de Young supérieur au premier module de Young en dehors de ladite phase d'acquisition.

Un autre mode de réalisation prévoit un dispositif d'imagerie ultrasonore comportant un ensemble de transducteurs ultrasonores et une couche de couplage acoustique revêtant ledit ensemble de transducteurs ultrasonores, dans lequel la couche de couplage acoustique est en un matériau polymère activable configuré pour présenter un premier module de Young lors d'une phase d'acquisition d'une image ultrasonore et un deuxième module de Young supérieur au premier module de Young en dehors de ladite phase d'acquisition.

Selon un mode de réalisation, la couche de couplage acoustique est en un polymère thermo-actif, photo-actif, électro-actif, ou un polymère activable sous l'effet d'un stimulus chimique ou mécanique.

Selon un mode de réalisation, la couche de couplage acoustique est en un polymère thermo-actif, le dispositif d'imagerie comportant un dispositif de chauffe configuré pour chauffer la couche de couplage acoustique lors d'une phase d'acquisition d'une image ultrasonore, et interrompre la chauffe en dehors de ladite phase d'acquisition.

Selon un mode de réalisation, la couche de couplage acoustique est en un polymère thermo-actif activable directement sous l'effet de la chaleur générée par un objet à imager, par exemple un doigt d'un utilisateur.

Selon un mode de réalisation, la couche de couplage acoustique est en un polymère thermo-actif activable pour présenter le premier module de Young lorsque sa température dépasse un seuil compris entre 25°C et 40°C.

Selon un mode de réalisation, la couche de couplage acoustique est en un matériau photo-actif dont le module de Young prend la première valeur sous l'effet d'une irradiation lumineuse à une première longueur d'onde A, et reprend la deuxième valeur sous l'effet d'une irradiation lumineuse à une deuxième longueur d'onde B, différente de A, ou lorsque l'irradiation à la première longueur d'onde A est interrompue, ou sous l'effet d'un autre stimulus.

Selon un mode de réalisation, le dispositif comprend une ou plusieurs premières sources lumineuses adaptées à émettre un rayonnement à la longueur d'onde A à travers la couche de couplage acoustique, et une ou plusieurs deuxièmes sources lumineuses adaptées à émettre un rayonnement à la longueur d'onde B à travers la couche de couplage acoustique.

Selon un mode de réalisation, le dispositif comprend en outre un circuit électronique d'alimentation et de contrôle de l'ensemble de transducteurs ultrasonores, lesdites une ou plusieurs premières et une ou plusieurs deuxièmes sources lumineuses étant commandées par le circuit électronique d'alimentation et de contrôle.

Selon un mode de réalisation, la couche de couplage acoustique est en un matériau électro-actif dont le module de Young prend la première valeur sous l'effet d'une polarisation électrique, et reprend la deuxième valeur en l'absence de ladite polarisation électrique.

Selon un mode de réalisation, le dispositif comprend en outre un circuit électronique d'alimentation et de contrôle de l'ensemble de transducteurs ultrasonores, le dispositif comprenant des première et deuxième électrodes en contact avec la couche de couplage acoustique et connectées au circuit électronique d'alimentation et de contrôle pour l'application de ladite polarisation électrique.

Selon un mode de réalisation, les transducteurs ultrasonores de l'ensemble de transducteurs ultrasonores sont des transducteurs CMUT ou PMUT, des transducteurs piézoélectriques ou piézocomposites, ou des transducteurs monocristal.

Selon un mode de réalisation, le dispositif est adapté à chauffer un objet à imager disposé sur la couche de couplage acoustique lors d'une phase d'acquisition d'une image ultrasonore.

### Brève description des dessins

Ces caractéristiques et avantages, ainsi que d'autres, seront exposés en détail dans la description suivante de modes de réalisation particuliers faite à titre non limitatif en relation avec les figures jointes parmi lesquelles :
la figure 1 représente de façon schématique un exemple d'un dispositif d'imagerie ultrasonore selon un mode de réalisation ;
la figure 2 représente de façon schématique un autre exemple d'un dispositif d'imagerie ultrasonore selon un mode de réalisation ;
la figure 3 représente de façon schématique un autre exemple d'un dispositif d'imagerie ultrasonore selon un mode de réalisation ;
la figure 4 représente de façon schématique un autre exemple d'un dispositif d'imagerie ultrasonore selon un mode de réalisation ;
la figure 5 représente de façon schématique un autre exemple d'un dispositif d'imagerie ultrasonore selon un mode de réalisation ;
la figure 6 représente de façon schématique un autre exemple d'un dispositif d'imagerie ultrasonore selon un mode de réalisation ;
la figure 7 représente de façon schématique un autre exemple d'un dispositif d'imagerie ultrasonore selon un mode de réalisation ; et
la figure 8 représente de façon schématique un autre exemple d'un dispositif d'imagerie ultrasonore selon un mode de réalisation.

### Description des modes de réalisation

De mêmes éléments ont été désignés par de mêmes références dans les différentes figures. En particulier, les éléments structurels et/ou fonctionnels communs aux différents modes de réalisation peuvent présenter les mêmes références et peuvent disposer de propriétés structurelles, dimensionnelles et matérielles identiques.

Par souci de clarté, seuls les étapes et éléments utiles à la compréhension des modes de réalisation décrits ont été représentés et sont détaillés. En particulier, la réalisation des transducteurs ultrasonores et des circuits électroniques de commande des dispositifs décrits n'a pas été détaillée, les modes de réalisation décrits étant compatibles avec les réalisations usuelles de ces éléments. En outre, les diverses applications que peuvent avoir les dispositifs décrits n'ont pas été détaillées, les modes de réalisation décrits étant compatibles avec toutes ou la plupart des applications usuelles des dispositifs d'imagerie ultrasonore, et en particulier des applications d'imagerie de parties du corps humain ou animal.

Sauf précision contraire, lorsque l'on fait référence à deux éléments connectés entre eux, cela signifie directement connectés sans éléments intermédiaires autres que des conducteurs, et lorsque l'on fait référence à deux éléments reliés (en anglais "coupled") entre eux, cela signifie que ces deux éléments peuvent être connectés ou être reliés par l'intermédiaire d'un ou plusieurs autres éléments.

Dans la description qui suit, lorsque l'on fait référence à des qualificatifs de position absolue, tels que les termes "avant", "arrière", "haut", "bas", "gauche", "droite", etc., ou relative, tels que les termes "dessus", "dessous", "supérieur", "inférieur", etc., ou à des qualificatifs d'orientation, tels que les termes "horizontal", "vertical", etc., il est fait référence sauf précision contraire à l'orientation des figures.

Sauf précision contraire, les expressions "environ", "approximativement", "sensiblement", et "de l'ordre de" signifient à 10 % près, de préférence à 5 % près.

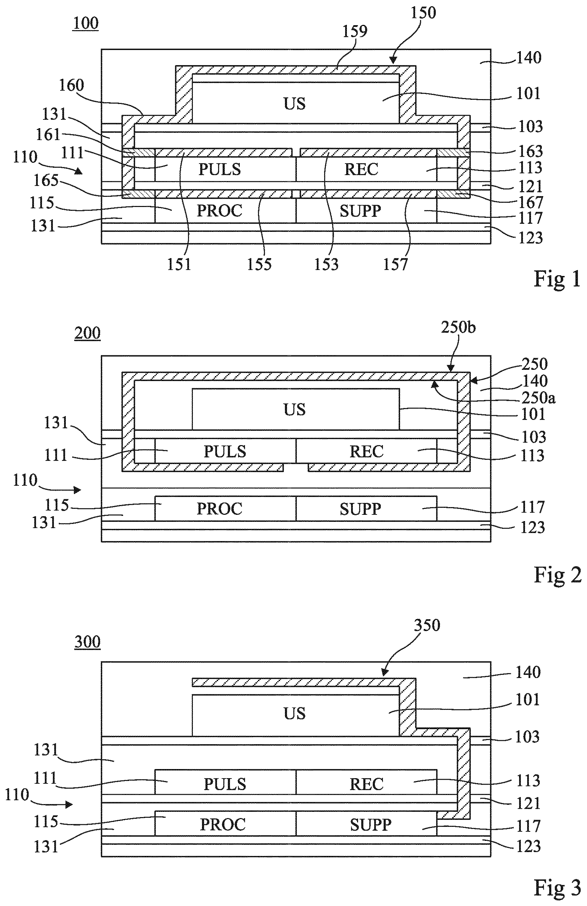

La figure 1 est une vue en coupe représentant de façon schématique un exemple d'un dispositif d'imagerie ultrasonore 100 selon un mode de réalisation.

Le dispositif 100 de la figure 1 comprend un ensemble 101 de transducteurs ultrasonores (US), par exemple disposés en matrice, en barrette, ou selon toute autre disposition. Les transducteurs de l'ensemble 101 sont par exemple des transducteurs de type CMUT (transducteurs ultrasonores capacitifs à membrane), des transducteurs de type PMUT (transducteurs piézoélectriques à membrane), des transducteurs à cristal, ou tout autre type de transducteurs ultrasonores, par exemple des transducteurs piézoélectriques ou piézocomposites, ou des transducteurs monocristal ("single crystal" en anglais).

Les transducteurs de l'ensemble 101 sont par exemple intégrés dans une puce monolithique, par exemple formée dans et sur un substrat semiconducteur, par exemple un substrat en silicium, ou encore dans et sur un substrat isolant, par exemple un substrat en verre (non détaillé sur la figure).

Dans l'exemple représenté, l'ensemble de transducteurs 101 est monté sur une carte de circuits imprimés 103. La carte de circuits imprimés 103 comprend par exemple un support en un matériau électriquement isolant, par exemple en plastique, et un ensemble de plages et pistes métalliques d'interconnexion (non détaillées sur la figure) formées sur au moins une face du support. Dans cet exemple, l'ensemble 101 de transducteurs ultrasonores est fixé et connecté électriquement à la face supérieure de la carte de circuits imprimés 103.

Le dispositif 100 de la figure 1 comprend en outre un circuit électronique d'alimentation et de contrôle 110.

Le circuit 110 comprend un circuit d'émission 111 (PULS) adapté à fournir des signaux électriques d'excitation aux transducteurs ultrasonores de l'ensemble 101 de façon à provoquer l'émission d'ondes ultrasonores par les transducteurs.

Le circuit 110 comprend en outre un circuit de réception 113 (REC) adapté à lire des signaux électriques de réponse générés par les transducteurs ultrasonores de l'ensemble 101 sous l'effet d'une onde ultrasonore reçue en provenance de l'objet à imager.

Le circuit 110 comprend de plus un circuit 115 (PROC) de traitement des signaux électriques émis par le circuit d'émission 111 et/ou reçus par le circuit de réception 113.

Le circuit 110 comprend de plus un circuit d'alimentation électrique 117 (SUPP). Le circuit 117 est notamment adapté à alimenter électriquement les circuits d'émission 111, de réception 113 et/ou de traitement 115.

Les circuits 111, 113, 115 et 117 sont par exemple intégrés dans une ou plusieurs puces de circuits intégrés. A titre d'exemple, les circuits 111, 113, 115 et 117 sont intégrés respectivement dans quatre puces de circuits intégrés distinctes. Les modes de réalisation décrits ne se limitent toutefois pas à ce cas particulier.

Les circuits 111, 113, 115 et 117 sont par exemple montés sur une ou plusieurs cartes de circuits imprimés. Dans l'exemple représenté, les circuits d'émission 111 et de réception 113 sont montés sur une carte de circuits imprimés 121, et les circuits de traitement 115 et d'alimentation 117 sont montés sur une autre carte de circuits imprimés 123. Plus particulièrement, dans cet exemple, les circuits d'émission 111 et de réception 113 sont fixés et connectés électriquement à la face supérieure de la carte de circuits imprimés 121, et les circuits de traitement 115 et d'alimentation 123 sont fixés et connectés électriquement à la face supérieure de la carte de circuits imprimés 123.

Dans l'exemple de la figure 1, l'ensemble de transducteurs ultrasonores 101 et le circuit électronique d'alimentation et de contrôle 110 sont intégrés de façon monolithique, par exemple dans un même boîtier (non visible sur la figure) de façon à former un module d'imagerie ultrasonore monolithique.

Dans cet exemple, l'ensemble 101 de transducteurs ultrasonores est disposé au-dessus du circuit électronique d'alimentation et de contrôle 110. Plus particulièrement, dans l'exemple représenté, la carte de circuits imprimés 103 est disposée au-dessus de l'ensemble comprenant la carte de circuit imprimés 121 et les circuits 111 et 113, qui est lui-même disposé au-dessus de l'ensemble comprenant la carte de circuit imprime 123 et les circuits 115 et 117. Autrement dit, les cartes de circuits imprimé 123, 121 et 103 sont empilées verticalement. Des éléments de fixation mécanique, non représentés, peuvent être prévus pour fixer mécaniquement les cartes de circuits imprimés les unes aux autres. De plus, des éléments de connexion électrique, non représentés, peuvent être prévus pour relier électriquement les cartes de circuits imprimés les unes aux autres.

Un matériau diélectrique de protection 131, par exemple une résine de protection, peut être disposé entre les cartes de circuits imprimés 123 et 121 et entre les cartes de circuits imprimés 121 et 103. A titre d'exemple, le matériau 131 comble entièrement les espaces libres entre la carte de circuits imprimés 123 et la carte de circuits imprimés 121 et entre la carte de circuits imprimés 121 et la carte de circuits imprimés 103. Le matériau de protection 131 peut en outre recouvrir la face inférieure de la carte de circuits imprimés 123, et la face supérieure de la carte de circuits imprimés 103 autour de l'ensemble 101 de transducteurs ultrasonores.

Le dispositif 100 de la figure 1 comprend en outre une couche de couplage 140, par exemple en un matériau polymère, par exemple électriquement isolant, revêtant la face supérieure de l'ensemble 101 de transducteurs ultrasonores. A titre d'exemple, la couche 140 est disposée sur et en contact avec la face supérieure de l'ensemble 101 de transducteurs ultrasonores. A titre de variante, la couche 140 est fixée sur la face supérieure de l'ensemble 101 de transducteurs par l'intermédiaire d'une couche adhésive, non représentée.

Dans l'exemple représenté, la couche 140 recouvre également la face supérieure de la carte de circuits imprimés 103 autour de l'ensemble 101 de transducteurs ultrasonores. Plus particulièrement, dans cet exemple, la couche 140 s'étend de façon continue sur toute la surface de la carte de circuits imprimés 103.

L'épaisseur de la couche de couplage 140 est par exemple comprise entre 100 µm et 5 mm, par exemple entre 500 µm et 1 mm.

La face supérieure de la couche 140 définit une surface de contact sur laquelle vient se poser un objet à imager, par exemple un ou plusieurs doigts d'un utilisateur, la paume d'un utilisateur, ou tout autre partie du corps humain ou animal que l'on souhaite imager.

La couche 140 a une fonction de couplage ultrasonore entre les transducteurs de l'ensemble 101 et l'objet à imager. En particulier, la couche 140 est de préférence relativement souple et déformable pour éviter la présence de lames d'air entre les transducteurs de l'ensemble 101 et l'objet à imager. De plus, la couche 140 a de préférence une impédance acoustique adaptée à celle de l'objet à imager, par exemple une impédance acoustique sensiblement égale à l'impédance acoustique de la peau. Ainsi, la couche 140 permet de maximiser le transfert d'énergie acoustique entre les transducteurs ultrasonores et l'objet à analyser.

La couche 140 peut en outre avoir une fonction de protection du dispositif et en particulier des transducteurs ultrasonores de l'ensemble 101. En particulier, la couche 140 présente de préférence une tenue mécanique relativement élevée pour ne pas se dégrader avec le temps et au contact des objets à imager.

Des expériences réalisées par les inventeurs ont montré que, pour certaines applications, par exemple pour des applications d'imagerie microvasculaire, la qualité des images acquises dépend fortement de la température de l'objet imagé. Par exemple, l'imagerie des faibles flux sanguins et des vaisseaux microscopiques situés aux extrémités des doigts de la main et du pied est difficile à réaliser car ces vaisseaux se situent aux extrémités les plus froides et peuvent subir une vasoconstriction et donc une diminution du flux sanguin.

Selon un aspect d'un premier mode de réalisation, on prévoit d'intégrer au dispositif d'imagerie un dispositif de chauffe adapté à chauffer l'objet à imager lors d'une phase d'acquisition d'une image ultrasonore. Ceci permet d'augmenter la température de l'objet pendant l'acquisition, et ainsi d'augmenter la qualité des images acquises, notamment pour des applications d'imagerie microvasculaire.

Dans l'exemple de la figure 1, le dispositif de chauffe 150 est un dissipateur thermique configuré pour collecter de la chaleur générée par le circuit électronique de contrôle et d'alimentation 110, et la conduire du côté de la face supérieure du dispositif, au-dessus de l'ensemble 101 de transducteurs ultrasonores.

Plus particulièrement, dans cet exemple, le dispositif de chauffe 150 comprend une première feuille ou couche métallique 151 recouvrant la face supérieure du circuit d'émission 111, une deuxième feuille ou couche métallique 153 recouvrant la face supérieure du circuit de réception 113, une troisième feuille ou couche métallique 155 recouvrant la face supérieure du circuit de traitement 115, et une quatrième feuille ou couche métallique 157 recouvrant la face supérieure du circuit d'alimentation 117. Chacune des feuilles métalliques 151, 153, 155 et 157 s'étend par exemple de façon continue sur toute la surface du circuit électronique 111, respectivement 113, respectivement 115, respectivement 117, sous-jacent.

Le dispositif de chauffe 150 de la figure 1 comprend en outre une feuille ou couche métallique 159 recouvrant la face supérieure de l'ensemble 101 de transducteurs ultrasonores. La feuille métallique 159 s'étend par exemple de façon continue au-dessus de toute la surface supérieure de l'ensemble 101 de transducteurs ultrasonores. A titre d'exemple, la feuille métallique 159 est noyée dans la couche de couplage 140, entre la face supérieure de l'ensemble 101 de transducteurs ultrasonores et la face supérieure de la couche 140. Autrement dit, la feuille métallique 159 recouvre une partie inférieure de la couche 140 et est recouverte par une partie supérieure de la couche 140. A titre de variante, la feuille métallique 159 est située sur et en contact avec la face supérieure de la couche de couplage.

Le dispositif de chauffe 150 de la figure 1 comprend en outre des régions métalliques 160 reliant thermiquement les feuilles métalliques 151, 153, 155 et 157 à la feuille métallique supérieure 159.

Dans l'exemple de la figure 1, le dispositif de chauffe 150 comprend en outre quatre interrupteurs thermiques 161, 163, 165, 167 reliant thermiquement respectivement les feuilles métalliques 151, respectivement 153, respectivement 155, respectivement 157, à la feuille métallique supérieure 159. Chacun des interrupteurs 161, 163, 165, 167 est adapté à, dans une première configuration, connecter thermiquement la feuille métallique 161, respectivement 163, respectivement 165, respectivement 167, à la feuille métallique supérieure 159, et, dans une deuxième configuration, isoler thermiquement la feuille métallique 161, respectivement 163, respectivement 165, respectivement 167, à la feuille métallique supérieure 159.

Les interrupteurs 161, 163, 165 et 167 sont par exemple des interrupteurs mécaniques, par exemple des bilames métalliques, adaptés à passer automatiquement de la première configuration à la deuxième configuration lorsque leur température dépasse un premier seuil prédéfini, et de la deuxième configuration à la première configuration lorsque leur température redescend sous un deuxième seuil prédéfini, égal au premier seuil ou inférieur au premier seuil (hystérésis). A titre de variante, les interrupteurs 161, 163, 165, 167 sont des interrupteurs commandés électriquement en fonction de mesures de températures réalisées au moyen d'un ou plusieurs capteurs de température du dispositif, non détaillés sur la figure. A titre de variante, les interrupteurs 161, 163, 165 et 167 peuvent être omis, les feuilles métalliques de collecte de chaleur 151, 153, 155 et 157 étant par exemple directement connectées thermiquement à la feuille métallique supérieure 159.

En fonctionnement, la chaleur produite par le circuit électronique de contrôle et d'alimentation 110 est collectée par les feuilles métalliques de collecte 151, 153, 155 et 157, et conduite vers la face supérieure du dispositif où elle est dissipée par la feuille métallique de dissipation 159.

Ceci permet de chauffer l'objet à imager pendant une phase d'acquisition d'une image ultrasonore.

Les interrupteurs 161, 163, 165, 167 permettent, le cas échéant, de stopper la totalité ou une partie du flux de chaleur transmis vers la feuille métallique supérieure 159 si la chaleur générée par le circuit électronique de contrôle et d'alimentation 110 est trop importante, de façon à prévenir des risques de brulure de l'utilisateur et/ou de dégradation de la couche de couplage 140 ou des transducteurs ultrasonores. Dans ce cas, le flux de chaleur peut éventuellement être redirigé vers un dissipateur auxiliaire, non représenté, disposé, par exemple, du côté de la face inférieure du dispositif.

Un avantage du dispositif 100 de la figure 1 est que le dispositif de chauffe 150 récupère la chaleur générée par le circuit électronique de contrôle et d'alimentation 110 pour chauffer l'objet à imager et ainsi améliorer la qualité des images acquises. Un autre avantage est que les couches métalliques assurent en outre une fonction de blindage des transducteurs ultrasonores, protégeant ces derniers contre d'éventuelles perturbations électromagnétiques.

La figure 2 est une vue en coupe représentant de façon schématique un autre exemple d'un dispositif d'imagerie ultrasonore 200 selon un mode de réalisation.

Le dispositif 200 de la figure 2 diffère du dispositif 100 de la figure 1 essentiellement en ce que, dans le dispositif 200, le dispositif de chauffe 150 de la figure 1 est remplacé par un dispositif de chauffe 250.

Le dispositif de chauffe 250 de la figure 2 est un module Peltier flexible. Le module Peltier 250 a la forme d'un ruban ou d'une nappe flexible et présente une première face 250a, appelée face froide, destinée à être placée contre un dispositif générant de la chaleur, et une deuxième face 250b, appelée face chaude, destinée à être tournée vers une zone d'évacuation de la chaleur.

Dans cet exemple, le module Peltier recouvre la surface supérieure de l'ensemble 101 de transducteurs ultrasonores, les bords du module Peltier étant repliés sous une partie du circuit électronique de contrôle et d'alimentation 110.

Plus particulièrement, dans l'exemple de la figure 2, les circuits d'émission 111 et de réception 113 sont fixés et connectés électriquement à la face inférieure de la carte de circuits imprimés 103, et les bords du module Peltier sont repliés sous la face inférieure des circuits 111 et 113 et au-dessus des circuits 115 et 117. Dans cet exemple, la carte de circuits imprimés 121 peut être omise.

A titre d'exemple, la partie supérieure du module Peltier, recouvrant l'ensemble 101 de transducteurs ultrasonores, est noyée dans la couche de couplage 140, entre la face supérieure de l'ensemble 101 de transducteurs ultrasonores et la face supérieure de la couche 140.

Dans cet exemple, au niveau de la partie supérieure du module Peltier 250, la face chaude 250b du module Peltier est tournée vers la face supérieure du dispositif d'imagerie, c'est-à-dire vers l'objet à imager. Au niveau des bords repliés du module Peltier 250, la face froide 250a du module Peltier est en contact avec la face inférieure des circuits électroniques 111 et 113.

En fonctionnement, le module Peltier 250 refroidit les circuits électroniques 111 et 113 et évacue la chaleur générée du côté de sa face chaude et en particulier du côté de la face supérieure du dispositif d'imagerie, sur laquelle est posé l'objet à imager.

Un avantage du dispositif 200 de la figure 2 est que le dispositif de chauffe 250 récupère la chaleur générée par le circuit électronique de contrôle et d'alimentation 110 pour chauffer l'objet à imager, et permet en outre de refroidir le circuit électronique de contrôle et d'alimentation 110.

La figure 3 est une vue en coupe représentant de façon schématique un autre exemple d'un dispositif d'imagerie ultrasonore 300 selon un mode de réalisation.

Le dispositif 300 de la figure 3 diffère du dispositif 100 de la figure 1 essentiellement en ce que, dans le dispositif 300, le dispositif de chauffe 150 de la figure 1 est remplacé par un dispositif de chauffe 350.

Le dispositif de chauffe 350 de la figure 3 est un tapis chauffant résistif comprenant une résistance métallique en serpentin, par exemple disposée entre deux feuilles d'un matériau électriquement isolant, par exemple un polyimide.

Le tapis chauffant 350 recouvre par exemple toute la surface supérieure de l'ensemble 101 de transducteurs ultrasonores. Le tapis chauffant 350 est par exemple connecté au circuit d'alimentation 117 pour son alimentation électrique.

Dans l'exemple de la figure 3, le tapis chauffant 350 est noyé dans la couche de couplage 140, entre la face supérieure de l'ensemble 101 de transducteurs ultrasonores et la face supérieure de la couche 140.

Un avantage du dispositif 350 de la figure 3 est que le dispositif de chauffe 350 peut être contrôlé précisément pour obtenir la température souhaitée au niveau de la face supérieure de la couche de couplage 140.

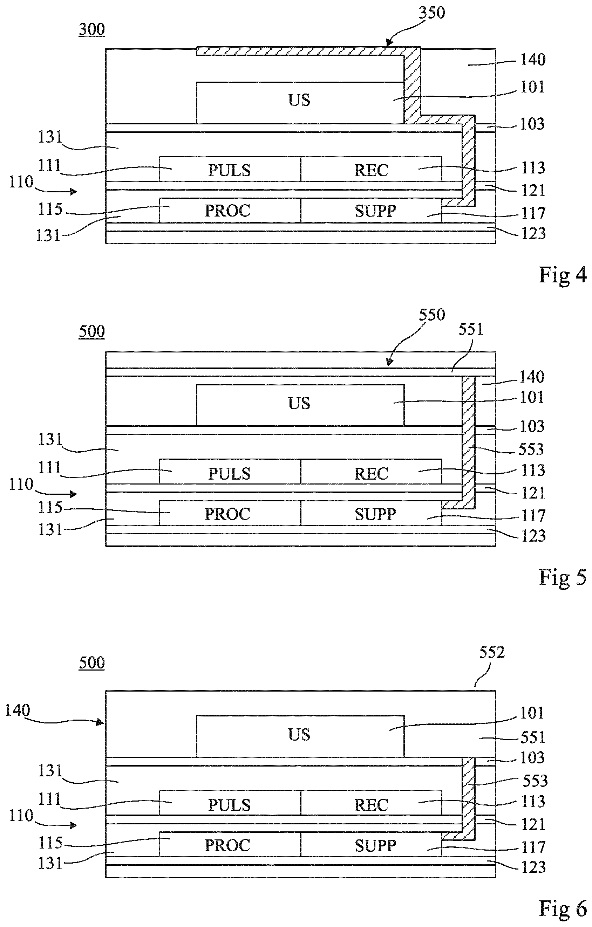

La figure 4 est une vue en coupe représentant de façon schématique une variante de réalisation du dispositif d'imagerie ultrasonore 300 de la figure 3.

Dans cette variante, le tapis chauffant 350 affleure au niveau de la face supérieure de la couche de couplage 140. Cette variante est moins favorable pour l'acquisition d'empreintes de peau car, le plan d'image à acquérir (la surface de la peau) est très proche des serpentins métalliques du tapis chauffant. Ainsi, l'empreinte des serpentins métalliques se superpose à l'image du doigt, ce qui dégrade la qualité de l'acquisition. Cette variante est en revanche adaptée pour l'acquisition d'une image dans un plan plus éloigné de la surface de contact du dispositif, par exemple une image microvasculaire, le tapis chauffant n'étant alors plus visible sur l'image acquise.

La figure 5 est une vue en coupe représentant de façon schématique un autre exemple d'un dispositif d'imagerie ultrasonore 500 selon un mode de réalisation.

Le dispositif 500 de la figure 5 diffère du dispositif 100 de la figure 1 essentiellement en ce que, dans le dispositif 500, le dispositif de chauffe 150 de la figure 1 est remplacé par un dispositif de chauffe 550.

Le dispositif de chauffe 550 comprend une couche chauffante 551 en un matériau polymère thermoélectrique, c'est-à-dire un matériau polymère adapté à générer de la chaleur sous l'effet d'une polarisation électrique.

La couche 551 est par exemple en un matériau à base de poly(3,4-éthylènedioxythiophène) (PEDOT), ou en un matériau polymère dans lequel sont adjointes des nanoparticules conductrices, par exemple des nanofils d'argent, permettant d'obtenir un niveau de conductivité générant un échauffement lors d'une mise sous contrainte électrique. A titre de variante, la couche 551 est en un oxyde conducteur transparent, par exemple de l'oxyde d'indium étain (ITO - de l'anglais "Indium Tin Oxide"), de l'oxyde d'indium étain dopé au fluor (FTO - de l'anglais "Fluorine doped Tin Oxide"), ou de l'oxyde de zinc dopé à l'aluminium (AZO - de l'anglais "Aluminium doped Zinc Oxide"). A titre de variante, la couche 551 est en un matériau à base de nanotubes de carbone ou de graphène.

Dans cet exemple, la couche chauffante 551 est intégrée dans la couche de couplage 140. Plus particulièrement, dans cet exemple, la couche 551 recouvre une partie inférieure de la couche de couplage 140 et est recouverte par une partie supérieure de la couche de couplage 140.

Dans cet exemple, l'épaisseur de la couche chauffante 551 est relativement mince par rapport à l'épaisseur totale de la couche de couplage 140.

A titre d'exemple, l'épaisseur de la couche chauffante 551 est comprise entre 50 et 200 nm, par exemple de l'ordre de 100 nm.

Le dispositif de chauffe 550 de la figure 5 comprend en outre des éléments de connexion électrique 553 reliant électriquement la couche chauffante 551 au circuit électronique d'alimentation 117 du dispositif.

La figure 6 est une vue en coupe représentant de façon schématique une variante de réalisation du dispositif d'imagerie ultrasonore 500 de la figure 5.

Dans cette variante, la couche chauffante 551 présente une épaisseur relativement importante, et constitue la majeure partie de l'épaisseur de la couche de couplage 140. La couche de couplage 140 peut en outre comprendre une couche électriquement isolante 552 relativement mince recouvrant la couche 551, pour prévenir tout risque d'électrocution de l'utilisateur.

Plus généralement, les dispositifs de chauffe 150, 250, 350 et 550 des dispositifs d'imagerie décrits en relation avec les figures 1, 2, 3, 4, 5 et 6 peuvent être remplacés par tout autre dispositif de chauffe adapté à chauffer l'objet à imager pendant une phase d'acquisition d'une image ultrasonore de l'objet, par exemple un dispositif à base de LED infrarouge.

Selon un aspect d'un second mode de réalisation, la couche de couplage 140 du dispositif d'imagerie est en un matériau polymère présentant des propriétés mécaniques et en particulier un module de Young modifiables sous l'effet d'un stimulus, par exemple un stimulus thermique (chauffage ou refroidissement), lumineux, électrique, chimique (eau, pH, ...), ou mécanique (pression ou impact). La couche de couplage 140 est configurée pour présenter un premier module de Young lors d'une phase d'acquisition d'une image ultrasonore, et un deuxième module de Young supérieur au premier module de Young en dehors de ladite phase d'acquisition. En d'autres termes, on prévoit de contrôler les propriétés mécaniques de la couche de couplage 140 de façon à améliorer les propriétés de couplage acoustique de la couche 140 pendant l'acquisition. Plus particulièrement, on prévoit de rendre la couche de couplage 140 plus conformable, par exemple plus molle ou souple pendant l'acquisition, de façon à améliorer le couplage acoustique entre les transducteurs et l'objet à analyser, et moins conformable, par exemple plus dure ou rigide en dehors de l'acquisition, pour renforcer les propriétés de protection mécanique conférées par la couche 140. A titre d'exemple, le module de Young de la couche de couplage 140 pendant l'acquisition d'une image ultrasonore est au moins 10 % plus faible, de préférence au moins 20 % plus faible, et de préférence au moins 50 % plus faible, qu'en dehors de l'acquisition, par exemple lorsque le dispositif d'imagerie est éteint. A titre d'exemple, le module de Young de la couche de couplage 140 est inférieur à 1,7 MPa et de préférence inférieur à 0,7 MPa pendant l'acquisition, et supérieur à 2,5 MPa et de préférence supérieur à 5,5 MPa en dehors de l'acquisition.

Selon un premier exemple de réalisation, la couche de couplage 140 est en un matériau polymère thermo-actif, c'est-à-dire un matériau polymère dont les propriétés mécaniques et en particulier le module de Young sont modifiés sous l'effet de la chaleur.

Dans ce cas, le dispositif de couplage peut comprendre un dispositif de chauffe adapté à chauffer la couche de couplage 140 à une température supérieure à la température ambiante, par exemple à une température supérieure à 25°C, de préférence supérieure à 30°C, de préférence supérieure à 35°C, de préférence supérieure à 40°C, pendant une phase d'acquisition d'une image ultrasonore, de manière à diminuer le module de Young de la couche 140. En dehors des phases d'acquisition, le dispositif de chauffe peut être interrompu de manière à ramener la couche de couplage 140 à la température ambiante et ainsi augmenter son module de Young.

Le dispositif de chauffe peut être identique ou similaire aux dispositifs décrits précédemment en relation avec les figures 1 à 6. Autrement dit, le premier mode de réalisation (chauffe de l'objet à imager pendant l'acquisition) et le premier exemple du second mode de réalisation (modification des propriétés mécaniques de la couche de couplage par chauffage pendant l'acquisition) peuvent être combinés.

A titre de variante, la chaleur utilisée pour réduire le module de Young de la couche de couplage pendant l'acquisition est uniquement la chaleur émise par l'objet à imager lui-même, par exemple un doigt d'un utilisateur, lorsque ce dernier est posé sur la face supérieure de la couche de couplage. Dans ce cas, le dispositif d'imagerie peut ne pas ne comprendre de dispositif de chauffe spécifique.

A titre d'exemple, la couche de couplage 140 est en un polymère thermo-actif à base de PTFE (polytétrafluoroéthylene), de PLA (polylactide), d'EVA (éthylene-vinyl acétate, de PCL (poly(ε-caprolactone), de tBA/PEGDMA (tert-butyl acrylate/de poly(éthylène glycol) diméthacrylate, de PU (polyuréthane), de PMMA (poly(méthyl méthacrylate), de polystyrène (PS), ou de silicone.

A titre de variante, un fonctionnement similaire peut être obtenu avec un polymère dont les propriétés mécaniques sont modifiées sous l'effet d'un refroidissement. Dans ce cas, le dispositif de chauffe peut être remplacé par un dispositif de refroidissement.

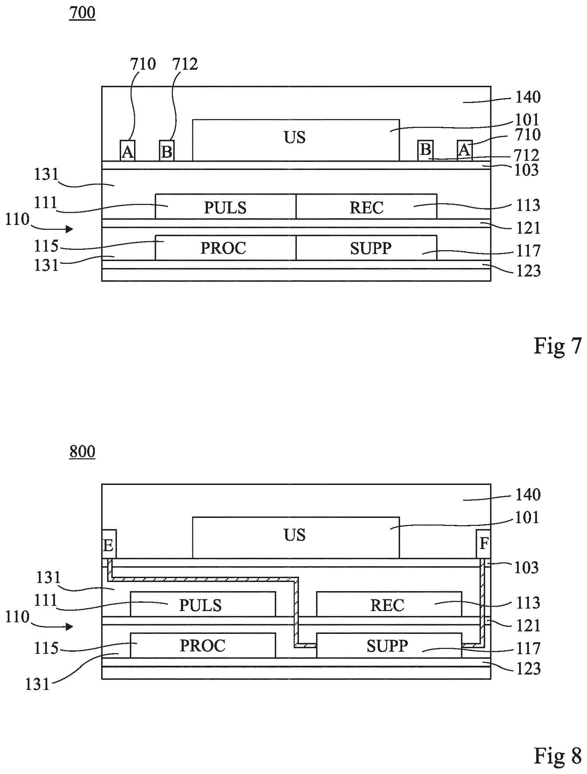

La figure 7 est une vue en coupe représentant de façon schématique un deuxième exemple d'un dispositif d'imagerie ultrasonore 700 selon le second mode de réalisation.

Dans cet exemple, la couche de couplage 140 est en un matériau polymère photo-actif, c'est-à-dire un matériau polymère dont les propriétés mécaniques et en particulier le module de Young sont modifiés sous l'effet d'un rayonnement lumineux.

Le dispositif 700 de la figure 7 comprend sensiblement les mêmes éléments que le dispositif 100 de la figure 1, à l'exception du dispositif de chauffe 150.

Dans cet exemple, la couche de couplage 140 est en un polymère photo-actif dont le module de Young prend une première valeur relativement faible sous l'effet d'une irradiation lumineuse à une première longueur d'onde A, et reprend une deuxième valeur relativement élevée sous l'effet d'une irradiation lumineuse à une deuxième longueur d'onde B, différente de A.

Les longueurs d'ondes A et B sont par exemple des longueurs d'ondes visibles ou infrarouges. Les modes de réalisation décrits ne se limitent toutefois pas à ce cas particulier.

Le dispositif 700 comprend une ou plusieurs sources lumineuses 710 adaptées à émettre un rayonnement à la longueur d'onde A à travers la couche de couplage 140, et une ou plusieurs sources lumineuses 712 adaptées à émettre un rayonnement à la longueur d'onde B à travers la couche de couplage 140. Les sources lumineuses 710 et 712 sont par exemple fixées et connectées sur la carte de circuits imprimés 103, à la périphérie de l'ensemble 101 de transducteurs ultrasonores. Les sources lumineuses 710 et 712 sont par exemple des diodes électroluminescentes (LED) adaptées à émettre respectivement à la longueur d'onde A et à la longueur d'onde B.

Le circuit électronique de contrôle et d'alimentation 110 est configuré pour, avant une phase d'acquisition d'une image ultrasonore, par exemple lorsqu'un objet à imager est détecté en vis-à-vis de l'ensemble 101 de transducteurs ultrasonores, activer la ou les sources lumineuses 710, de façon à rendre la couche de couplage 140 relativement molle et améliorer ses propriétés de couplage acoustique, puis, après l'acquisition, activer la ou les sources lumineuses 712 de façon à rendre la couche de couplage 140 relativement dure et améliorer ses propriétés de protection mécanique.

Par exemple, lorsque l'utilisateur pose un doigt sur la face supérieure de la couche 140 en vue d'une capture d'empreinte digitale, le circuit 110 active l'émission à la longueur d'onde A ce qui permet de relâcher le stress et permet à la couche 140 de s'adapter à la morphologie du doigt. A la fin de la capture, le polymère reprend sa forme lisse (autrement dit, la face supérieure de la couche 140 redevient sensiblement plane) et on active l'émission à la longueur d'onde B ce qui permet de rigidifier le polymère et de maintenir la couche 140 dans cet état.

De façon avantageuse, l'exemple de réalisation de la figure 7 peut être adapté à un dispositif d'imagerie photo-acoustique, c'est-à-dire un dispositif dans lequel, lors de l'acquisition d'une image ultrasonore, de la lumière modulée est émise en direction de l'objet à imager. Dans ce cas, la même source lumineuse peut être utilisée pour émettre la lumière modulée lors de l'acquisition et ramollir le polymère de couplage de la couche 140 (longueur d'onde A).

A titre de variante, le retour à l'état initial (module de Young relativement élevé) peut être obtenu simplement par interruption de l'irradiation à la longueur d'onde A, par chauffage ou refroidissement, ou sous l'effet d'un autre stimulus. Dans ce cas, les sources lumineuses émettant à la longueur d'onde B peuvent être omises.

A titre d'exemple, la couche de couplage 140 est en un polymère photo-actif du comprenant des groupements de type diazo, des dérivés d'acrylates, des dérivés cinnamiques, des dérivés furaniques ou autres groupements connus pour leurs propriétés photosensibles. La personne du métier sera à même de choisir le groupement approprié en fonction de sa compatibilité chimique avec le polymère de couplage de la couche 140 et des propriétés de photo-activation souhaitées.

La figure 8 est une vue en coupe représentant de façon schématique un troisième exemple d'un dispositif d'imagerie ultrasonore 800 selon le second mode de réalisation.

Dans cet exemple, la couche de couplage 140 est en un matériau polymère électro-actif, c'est-à-dire un matériau polymère dont les propriétés mécaniques et en particulier le module de Young sont modifiés sous l'effet d'une polarisation électrique, par exemple sous l'effet d'une tension électrique.

Le dispositif 800 de la figure 8 comprend sensiblement les mêmes éléments que le dispositif 100 de la figure 1, à l'exception du dispositif de chauffe 150.

Dans cet exemple, la couche de couplage 140 est en un polymère électro-actif dont le module de Young prend une première valeur relativement faible sous l'effet d'une polarisation électrique, par exemple une tension électrique, appliquée entre des électrodes E et F en contact avec le polymère, et une deuxième valeur relativement élevée en l'absence de ladite polarisation électrique.

Les électrodes E et F sont par exemple en contact avec le polymère de la couche de couplage 140 au voisinage respectivement de deux bords latéraux opposés de la couche 140.

Les électrodes E et F sont par exemple reliées au circuit électronique d'alimentation 117 du dispositif pour l'application de la polarisation électrique.

Le circuit électronique de contrôle et d'alimentation 110 est configuré pour, avant une phase d'acquisition d'une image ultrasonore, par exemple lorsqu'un objet à imager est détecté en vis-à-vis de l'ensemble 101 de transducteurs ultrasonores, appliquer la polarisation électrique de façon à rendre la couche de couplage 140 relativement molle et améliorer ses propriétés de couplage acoustique, puis, après l'acquisition, désactiver la polarisation électrique de façon à rendre la couche de couplage 140 relativement dure et améliorer ses propriétés de protection mécanique.

Par exemple, lorsque l'utilisateur pose un doigt sur la face supérieure de la couche 140 en vue d'une capture d'empreinte digitale, le circuit 110 active la polarisation électrique de la couche 140 ce qui permet de relâcher le stress et permet à la couche 140 de s'adapter à la morphologie du doigt. A la fin de la capture, le polymère reprend sa forme lisse (autrement dit, la face supérieure de la couche 140 redevient sensiblement plane) et on désactive la polarisation électrique ce qui permet de rigidifier le polymère et de maintenir la couche 140 dans cet état.

A titre d'exemple, la couche de couplage 140 est en un polymère électro-actif de la famille des polyacrylates, polyuréthanes, latex, caoutchoucs naturels, silicones, copolymères butadiène-acrylonitrile, polymères piézoélectriques par exemple type PVDF (polyfluorure de vinylidène), ou son copolymère P(VDF-TrFE) (fluorure de vinylidène et trifluoroéthylène) .

Divers modes de réalisation et variantes ont été décrits. La personne du métier comprendra que certaines caractéristiques de ces divers modes de réalisation et variantes pourraient être combinées, et d'autres variantes apparaîtront à la personne du métier. En particulier, les modes de réalisation décrits ne se limitent pas aux exemples de matériaux et de dimensions mentionnés dans la présente description.

En outre, la personne du métier saura combiner les différents exemples de réalisation décrits ci-dessus en fonction des besoins de l'application visée. En particulier, la personne du métier saura combiner les premier et deuxième modes de réalisation décrits ci-dessus pour bénéficier à la fois de l'avantage lié au chauffage de l'objet à imager lors de l'acquisition et de l'adaptation des propriétés mécaniques de la couche de couplage sous l'effet d'un stimulus. En particulier, les exemples de réalisation des figures 7 et 8 peuvent être combinés avec les exemples de réalisation des figures 1, 2, 3, 4, 5 ou 6.

Par ailleurs, bien que l'on ait décrit ci-dessus uniquement des exemples d'application à des dispositifs d'imagerie ultrasonore, la personne du métier saura, à partir des indications de la présente description, adapter les modes de réalisation décrits à d'autres dispositifs d'émission et/ou de réception d'ultrasons, par exemple des dispositifs de traitement du corps humain ou animal par émission d'ultrasons, ou encore des dispositifs de contrôle non destructif par ultrasons non nécessairement appliqués au corps humain ou animal.

Enfin, la mise en oeuvre pratique des modes de réalisation et variantes décrits est à la portée de la personne du métier à partir des indications fonctionnelles données ci-dessus.

## Revendications

1. Dispositif d'imagerie ultrasonore (100 ; 200 ; 300 ; 500) comportant un ensemble (101) de transducteurs ultrasonores, une couche de couplage acoustique (140) revêtant ledit ensemble (101) de transducteurs ultrasonores, **caractérisé en ce qu'**il comprend un dispositif de chauffe (150 ; 250 ; 350 ; 550) adapté pour chauffer la couche de couplage acoustique (140) et un objet à imager disposé sur la couche de couplage acoustique (140) pendant une phase d'acquisition d'une image ultrasonore.

2. Dispositif (100 ; 200 ; 300 ; 500) selon la revendication 1, comprenant en outre un circuit électronique (110) d'alimentation et de contrôle.

3. Dispositif (100 ; 200) selon la revendication 2, dans lequel le dispositif de chauffe (150 ; 250) est adapté à récupérer de la chaleur générée par le circuit électronique (110) d'alimentation et de contrôle et à dissiper tout ou partie de cette chaleur dans la couche de couplage acoustique (140), au-dessus de l'ensemble (101) de transducteurs ultrasonores.

4. Dispositif (100) selon la revendication 3, dans lequel le dispositif de chauffe (150) comprend une ou plusieurs feuilles métalliques (151, 153, 155, 157, 159) agencées pour conduire la chaleur générée par le circuit électronique (110) d'alimentation et de contrôle et dissiper tout ou partie de cette chaleur dans la couche de couplage acoustique (140), au-dessus de l'ensemble (101) de transducteurs ultrasonores.

5. Dispositif (100) selon la revendication 4, dans lequel le dispositif de chauffe (150) comprend en outre un ou plusieurs interrupteurs (161, 163, 165, 167) configurés pour interrompre le transfert de chaleur vers la couche de couplage acoustique (140) si une température interne du dispositif excède un seuil prédéterminé.

6. Dispositif (200) selon la revendication 3, dans lequel le dispositif de chauffe (250) comprend un module Peltier flexible dont une face froide (250a) est tournée vers le circuit électronique d'alimentation et de contrôle (110) et dont une face chaude (250b) est tournée vers l'objet à imager.

7. Dispositif (300) selon la revendication 1 ou 2, dans lequel le dispositif de chauffe (350) comprend un tapis de chauffe comprenant une résistance métallique en serpentin, disposé entre l'ensemble (101) de transducteurs ultrasonores et l'objet à imager.

8. Dispositif (300) selon la revendication 7, dans lequel le tapis de chauffe est noyé dans la couche de couplage acoustique (140).

9. Dispositif (300) selon la revendication 7, dans lequel le tapis de chauffe recouvre la couche de couplage acoustique (140).

10. Dispositif (500) selon la revendication 1 ou 2, dans lequel le dispositif de chauffe (550) comprend une couche chauffante (551) en un matériau polymère thermoélectrique, intégrée à la couche de couplage acoustique (140).

11. Dispositif (500) selon la revendication 10 dans son rattachement à la revendication 2, dans lequel la couche chauffante (551) est connectée électriquement au circuit électronique d'alimentation et de contrôle (110) par des éléments de connexion (553).

12. Dispositif (100 ; 200 ; 300 ; 500) selon l'une quelconque des revendications 1 à 11, dans lequel les transducteurs ultrasonores de l'ensemble (101) de transducteurs ultrasonores sont des transducteurs CMUT ou PMUT, des transducteurs piézoélectriques ou piézocomposites, ou des transducteurs monocristal.

13. Dispositif selon l'une quelconque des revendications 1 à 12, dans lequel la couche de couplage acoustique (140) comprend au moins une couche en un matériau polymère activable configuré pour présenter un premier module de Young lors d'une phase d'acquisition d'une image ultrasonore et un deuxième module de Young supérieur au premier module de Young en dehors de ladite phase d'acquisition.

## Patentansprüche

1. Eine Ultraschall-Bildgebungseinrichtung (100; 200; 300; 500), die Folgendes aufweist:
eine Anordnung (101) von Ultraschall-Wandlern,
eine akustische Kopplungsschicht (140), die die Anordnung (101) von Ultraschall-Wandlern beschichtet,
**dadurch gekennzeichnet, dass** sie eine Heizeinrichtung (150; 250; 350; 550) aufweist, die zum Erwärmen der akustischen Kopplungsschicht (140) und
eines auf der akustischen Kopplungsschicht (140) angeordneten abzubildenden Objekts während einer Phase der Erfassung eines Ultraschallbildes eingerichtet ist.

2. Die Einrichtung (100; 200; 300; 500) nach Anspruch 1, ferner eine elektronische Stromversorgungs- und Steuerschaltung (110) aufweisend.

3. Die Einrichtung (100; 200) nach Anspruch 2, wobei die Heizeinrichtung (150; 250) zum Sammeln von durch die elektronische Stromversorgungs- und Steuerschaltung (110) erzeugter Wärme und zum Ableiten der gesamten oder eines Teils dieser Wärme in die akustische Kopplungsschicht (140) oberhalb der Anordnung (101) von Ultraschall-Wandlern eingerichtet ist.

4. Die Einrichtung (100) nach Anspruch 3, wobei die Heizeinrichtung (150) eine oder eine Vielzahl von Metallfolien (151, 153, 155, 157, 159) aufweist, die zum Leiten der durch die elektronische Stromversorgungs- und Steuerschaltung (110) erzeugten Wärme und zum Ableiten der gesamten oder eines Teils dieser Wärme in die akustische Kopplungsschicht (140) oberhalb der Anordnung (101) von Ultraschall-Wandlern angeordnet sind.

5. Die Einrichtung (100) nach Anspruch 4, wobei die Heizeinrichtung (150) ferner einen oder eine Vielzahl von Schaltern (161, 163, 165, 167) aufweist, die zum Unterbrechen der Wärmeübertragung zu der akustischen Kopplungsschicht (140) eingerichtet sind, falls eine Innentemperatur der Einrichtung einen vorbestimmten Schwellenwert überschreitet.

6. Die Einrichtung (200) nach Anspruch 3, wobei die Heizeinrichtung (250) ein flexibles Peltier-Modul aufweist, das eine kalte Oberfläche (250a), die der elektronischen Stromversorgungs- und Steuerschaltung (110) zugewandt ist, und eine heiße Oberfläche (250b), die dem abzubildenden Objekt zugewandt ist, hat.

7. Die Einrichtung (300) nach Anspruch 1 oder 2, wobei die Heizeinrichtung (350) eine Heizmatte aufweist, die einen schlangenförmigen Metallwiderstand aufweist, der zwischen der Anordnung (101) von Ultraschall-Wandlern und dem abzubildenden Objekt angeordnet ist.

8. Die Einrichtung (300) nach Anspruch 7, wobei die Heizmatte in die akustische Kopplungsschicht (140) eingebettet ist.

9. Die Einrichtung (300) nach Anspruch 7, wobei die Heizmatte die akustische Kopplungsschicht (140) abdeckt.

10. Die Einrichtung (500) nach Anspruch 1 oder 2, wobei die Heizeinrichtung (550) eine Heizschicht (551) aufweist, die aus einem thermoelektrischen Polymermaterial hergestellt ist, das in die akustische Kopplungsschicht (140) integriert ist.

11. Die Einrichtung (500) nach Anspruch 10 in Abhängigkeit von Anspruch 2, wobei die Heizschicht (551) durch Verbindungselemente (553) elektrisch mit der elektronischen Stromversorgungs- und Steuerschaltung (110) verbunden ist.

12. Die Einrichtung (100; 200; 300; 500) nach einem der Ansprüche 1 bis 11, wobei die Ultraschall-Wandler der Anordnung (101) von Ultraschall-Wandlern CMUT- oder PMUT-Wandler, piezoelektrische oder piezokomposite Wandler oder Einkristall-Wandler sind.

13. Die Einrichtung nach einem der Ansprüche 1 bis 12, wobei die akustische Kopplungsschicht (140) wenigstens eine Schicht aufweist, die aus einem aktivierbaren Polymermaterial hergestellt ist, das eingerichtet ist, dass sie Folgendes hat:
ein erstes Elastizitätsmodul während einer Erfassungsphase eines Ultraschallbildes und ein zweites Elastizitätsmodul, das größer als das erste Elastizitätsmodul ist, und zwar außerhalb der genannten Erfassungsphase.

## Claims

1. Ultrasound imaging device (100; 200; 300; 500) comprising an assembly (101) of ultrasound transducers, an acoustic coupling layer (140) coating said assembly (101) of ultrasound transducers, **characterized in that** it comprises a heating device (150; 250; 350; 550) adapted to heating the acoustic coupling layer (140) and an object to be imaged arranged on the acoustic coupling layer (140) during a phase of acquisition of an ultrasound image.

2. Device (100; 200; 300; 500) according to claim 1, further comprising an electronic power supply and control circuit (110).

3. Device (100; 200) according to claim 2, wherein the heating device (150; 250) is adapted to collecting heat generated by the electronic power supply and control circuit (110) and to dissipating all or part of this heat into the acoustic coupling layer (140), above the assembly (101) of ultrasound transducers.

4. Device (100) according to claim 3, wherein the heating device (150) comprises one or a plurality of metal foils (151, 153, 155, 157, 159) arranged to conduct the heat generated by the electronic power and control circuit (110) and to dissipate all or part of this heat into the acoustic coupling layer (140), above the assembly (101) of ultrasound transducers.

5. Device (100) according to claim 4, wherein the heating device (150) further comprises one or a plurality of switches (161, 163, 165, 167) configured to interrupt the heat transfer towards the acoustic coupling layer (140) if an inner temperature of the device exceeds a predetermined threshold.

6. Device (200) according to claim 3, wherein the heating device (250) comprises a flexible Peltier module, having a cold surface (250a) facing the electronic power supply and control circuit (110) and having a hot surface (250b) facing the object to be imaged.

7. Device (300) according to claim 1 or 2, wherein the heating device (350) comprises a heating mat comprising a serpentine metal resistor, arranged between the assembly (101) of ultrasound transducers and the object to be imaged.

8. Device (300) according to claim 7, wherein the heating mat is embedded in the acoustic coupling layer (140).

9. Device (300) according to claim 7, wherein the heating mat covers the acoustic coupling layer (140).

10. Device (500) according to claim 1 or 2, wherein the heating device (550) comprises a heating layer (551) made of a thermoelectric polymer material, integrated to the acoustic coupling layer (140).

11. Device (500) according to claim 10 as dependent on claim 2, wherein the heating layer (551) is electrically connected to the electronic power supply and control circuit (110) by connection elements (553).

12. Device (100; 200; 300; 500) according to any of claims 1 to 11, wherein the ultrasound transducers of the assembly (101) of ultrasound transducers are CMUT or PMUT transducers, piezoelectric, or piezocomposite transducers, or single-crystal transducers.

13. Device according to any of claims 1 to 12, wherein the acoustic coupling layer (140) comprises at least one layer made of an activatable polymer material configured to have a first Young's modulus during a phase of acquisition of an ultrasound image and a second Young's modulus greater than the first Young's modulus outside of said acquisition phase.
